# EUROPEAN PATENT APPLICATION

(11) **EP 1 348 432 A1**
(43) Date of publication of application: **01.10.2003**
(21) Application number: 01273183.2
(22) Date of filing: 17.12.2001
(51) Int. Cl.: A61K 31/41, A61K 9/08, A61K 47/40, C07D 293/12

(54) **CYCLODEXTRIN-CONTAINING PHARMACEUTICAL PREPARATION**

(30) Priority: 04.01.2001 JP 2001000247; 24.01.2001 JP 2001016022
(71) Applicant: DAIICHI PHARMACEUTICAL CO., LTD., Chuo-ku, Tokyo 103-8234 (JP)
(72) Inventor: SUZUKI, N., c/o Daiichi Pharmaceutical Co., Ltd, Tokyo 134-8630 (JP); NAGASE, Y., c/o Daiichi Pharmaceutical Co., Ltd, Tokyo 134-8630 (JP); YAMAUCHI, H., c/o Daiichi Pharmaceutical Co., Ltd, Osaka Factory, 4-38, Aketacho (JP)
(74) Representative: Kinzebach, Werner, Dr.
(86) International application number: JP0111048
(87) International publication number: WO02055076

(57) **Abstract**

The present invention can provide the following which is a transparent aqueous solution of a sufficient amount of ebselen and usable as an injection. A water-based preparation or aqueous solution comprising ebselen and cyclodextrin. An injection comprising the aqueous solution. An intravenous drip infusion comprising the aqueous solution. A process for producing an aqueous solution containing ebselen and cyclodextrin, which comprises dissolving ebselen in a water-miscible organic solvent while separately dissolving a cyclodextrin in a water solvent, mixing both the solutions, then drying the mixture, and mixing the resulting dried product with a water solvent. A dried preparation comprising ebselen and cyclodextrin. A process for producing a solution containing ebselen and cyclodextrin, which comprises dissolving ebselen in a water-miscible organic solvent while separately dissolving cyclodextrin in a water solvent, and mixing both the solutions.

## Description

### TECHNICAL FIELD

The present invention relates to an aqueous liquid medicine and an aqueous solution comprising ebselen and cyclodextrin, a process for producing the same, a dried product and a dried preparation obtained by dissolving ebselen in an organic solvent while separately dissolving cyclodextrin in a water solvent, mixing both the solutions and drying the mixture, and an injection and an intravenous drip infusion comprising ebselen and cyclodextrin.

### BACKGROUND ART

The chemical name of ebselen is 2-phenyl-1,2-benzisoselenazole-3(2H)-one, which is a compound shown in the following chemical structural formula (I):

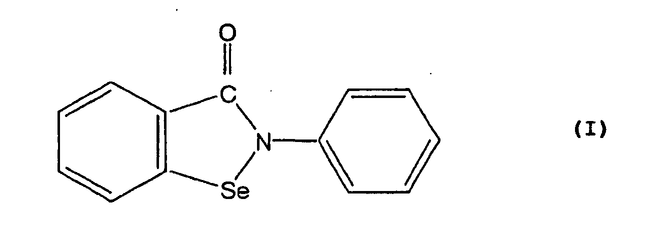

Ebselen is a known compound with known pharmacological effects including therapeutic effects on cerebral disorders such as cerebral infarction (JP-B-5-88684) (the term "JP-B" as used herein means an "examined Japanese patent publication") .

On the other hand, cyclodextrin is used widely as an agent for dissolving compounds having a very low solubility in water, and the known method of dissolving compounds having a very low solubility in water by use of cyclodextrin includes:.
1) Lyophilization method
   Reference: M. Kurozumi, N. Nambu, T. Nagai, Chem. Pharm. Bull., 23, 3062 (1975)
2) Solvent evaporation method
   Reference: M. Tsuruoka, et al., Yakugaku Zasshi (Journal of the Pharmaceutical Society of Japan), 101, 360 (1981)
3) Co-precipitation method (1) using ethanol
   Reference: M. Tsuruoka, et al., Yakugaku Zasshi, 101, 360 (1981)
4) Co-precipitation method (2) using ether
   Reference: M. Kurozumi, N. Nambu, T. Nagai, Chem. Pharm. Bull. , 23, 3062 (1975)
5) Ethanol method (a method of using ethanol as a dissolution assistant)
   Reference: J. Pitha, et al., Int. J. Pharm., 80, 253 (1992)

Ebselen is a compound having a very low solubility in water, and for preparing an aqueous solution thereof by increasing its solubility, various methods such as conversion thereof into fine particles (JP-B-7-13016), encapsulation into liposome (JP-B-8-30064) etc. have been taken, but an aqueous solution having ebselen dissolved sufficiently therein at a concentration usable as an injection, that is, a pharmaceutical injection is still not obtained.

### DISCLOSURE OF THE INVENTION

To obtain an aqueous solution having ebselen dissolved sufficiently therein, the present inventors arrived at a cyclodextrin preparation of this compound, and employed the known processes described above to produce the pharmaceutical preparation, but the desired preparation could not be obtained. Under these circumstances, the present inventors found that an aqueous solution having ebselen dissolved sufficiently therein could be obtained for the first time by dissolving ebselen in a water-miscible organic solvent while separately dissolving cyclodextrin in a water solvent, mixing both the solutions, drying the mixture and mixing the resulting dried product with a water solvent, and the present invention was thereby completed.

The present invention comprises the following constitutions:
(1) An aqueous liquid medicine comprising ebselen and cyclodextrin.
(2) An aqueous solution comprising ebselen and cyclodextrin.
(3) The aqueous solution according to item (2) above, wherein the cyclodextrin is a β-cyclodextrin sulfobutyl ether sodium salt or hydroxypropyl-β-cyclodextrin.
(4) The aqueous solution according to item (2) or (3) above,
   wherein the molar ratio of cyclodextrin and ebselen is from 1 : 2 to 1 : 50.
(5) An injection comprising an aqueous solution containing ebselen and cyclodextrin.
(6) The injection according to item (5) above, wherein the cyclodextrin is a β-cyclodextrin sulfobutyl ether sodium salt or hydroxypropyl-β-cyclodextrin.
(7) An intravenous drip infusion comprising an aqueous solution containing ebselen and cyclodextrin.
(8) The intravenous drip infusion according to item (7) above, wherein the cyclodextrin is a β-cyclodextrin sulfobutyl ether sodium salt or hydroxypropyl-β-cyclodextrin.
(9) A process for producing an aqueous solution containing ebselen and cyclodextrin, which comprises dissolving ebselen in a water-miscible organic solvent while separately dissolving cyclodextrin in a water solvent, mixing both the solutions, then drying the mixture, and mixing the resulting dried product with a water solvent.
(10) The process according to item (9) above, wherein the water-miscible organic solvent is ethanol, methanol, or a mixture of methanol and chloroform.
(11) A dried preparation comprising ebselen and cyclodextrin.
(12) The dried preparation according to item (11) above, wherein the cyclodextrin is a β-cyclodextrin sulfobutyl ether sodium salt or hydroxypropyl-β-cyclodextrin.
(13) A process for producing a solution containing ebselen and cyclodextrin, which comprises dissolving ebselen in a water-miscible organic solvent while separately dissolving cyclodextrin in a water solvent, and mixing both the solutions.

The present invention relates to an aqueous liquid medicine and an aqueous solution mainly containing ebselen and cyclodextrin.

Cyclodextrin usable in the present invention includes β-cyclodextrin sulfobutyl ether and salts thereof, hydroxypropyl-β-cyclodextrin, α-cyclodextrin (referred to hereinafter as α-CyD) , β-cyclodextrin (referred to hereinafter as β-CyD), γ-cyclodextrin (referred to hereinafter as γ-CyD) etc., among which β-cyclodextrin sulfobutyl ether and salts thereof as well as hydroxypropyl-β-cyclodextrin can be mentioned as preferable examples.

β-Cyclodextrin sulfobutyl ether is a substance in which hydroxyl groups at the 2-, 3- and 6-positions in glucopyranose constituting β-cyclodextrin have been substituted suitably with sulfobutyloxy groups, and the salts of this substance are typically those in which a hydroxysulfonyl group (HOSO₂-) of the substance forms a salt with an alkali metal such as sodium or an alkaline earth metal such as calcium. β-Cyclodextrin is a substance having 7 glucopyranoses bound in a cyclic form via α-1,4-linkages, and thus one molecule of β-cyclodextrin has 21 hydroxyl groups in total derived from the 2-, 3- and 6-positions in the glucopyranoses constituting β-cyclodextrin, among which preferably about 7 hydroxyl groups have been substituted with sulfobutyloxy groups and simultaneously the hydroxysulfonyl group (HOSO₂-) has formed preferably a sodium salt, and such substance includes e.g. CAPTISOL^{TM}.

Hydroxypropyl-β-cyclodextrin refers to a substance in which the 21 hydroxyl groups in β-cyclodextrin have been substituted suitably with 2-hydroxypropyloxy groups, and usually this substance is preferably the one in which out of the 21 hydroxyl groups, about 4 to 8 hydroxyl groups have been substituted with 2-hydroxypropyloxy groups and particularly preferably the one having a degree of substitution of about 4 to 5, and such substance include e.g. Celdex HP-β-CD^{TM} with a degree of substitution of 4.6-7.6.

Hereinafter, a process for producing the aqueous solution of the present invention is described.

Ebselen is dissolved in an organic solvent miscible with water, while cyclodextrin is dissolved in a water solvent, and thus ebselen is previously dissolved transparently in a water-miscible organic solvent such as lower alcohol (ethanol, methanol etc.) or a mixed solvent of chloroform and lower alcohol, while cyclodextrin is dissolved in a water solvent, and then the respective solutions are mixed whereby a transparent and uniform solution can be obtained.

The water solvent used is water alone or water having various additives dissolved therein, such as inorganic or organic salts such as sodium chloride, calcium chloride and citrates, sugars such as mannitol, glucose and lactose, surfactants such as Polysorbate 80, anesthetics such as procaine hydrochloride and polyols such as glycerin. The amounts of these additives may be used in a conventional range. To permit both ebselen and cyclodextrin to be dissolved more transparently in the resulting mixed solution, the proportion of organic solvent : water solvent is preferably from 2 : 1.2 to 2 : 1.3 (v/v). The proportion (molar ratio) of ebselen and cyclodextrin in the resulting mixed solution is usually from 1 : 2 to 1 : 50 (molar ratio), preferably from 1 : 5 to 1 : 40 (molar ratio) and more preferably from 1 : 9 to 1 : 30 (molar ratio).

The above-described additives such as inorganic salts, sugars, surfactants, anesthetics and co-solvents may be added to the resulting mixed solution for the purpose of rendering it isotonic or stabilizing it. These additives may be used in amounts in an ordinary range.

For preparation of the desired injection, the mixed solution obtained in the manner described above may be subjected to filtration or heat sterilization. Filtration and heat sterilization may be conducted in a usual manner.

The mixed solution prepared in this manner is introduced as necessary to a vial or an ampoule under aseptic conditions, followed by removing the water-miscible solvent and water by an evaporator or by drying means represented by spray drying and lyophilization to usually give a powdery dried product or a dried pharmaceutical preparation. The means such as spray drying and lyophilization may follow conventional methods. The resulting dried product can also be introduced depending on the case into a vial or an ampoule under aseptic conditions to give a dried pharmaceutical preparation.

When the dried product thus obtained is mixed with a water solvent, it is easily dissolved therein so that ebselen having a very low solubility in water can be solubilized transparently at a sufficient concentration also usable as an injection. The water solvent used includes water alone or those water solvents having inorganic salts, sugars, surfactants, anesthetics and co-solvents dissolved therein for the purpose of rendering the solution isotonic or stabilizing it, and these water solvents may have been subjected as necessary to the above-described filtration or heat sterilization. These additives may be used in amounts in an ordinary range. The concentration of ebselen in the finally obtained aqueous solution containing ebselen and cyclodextrin is not particularly limited, and can be in the range of usually 0.01 mg/ml to 100 mg/ml, preferably 0.5 mg/ml to 50 mg/ml, and the concentration of cyclodextrin therein is not particularly limited either, and can be in the range of usually 0.7 mg/ml to 7 g/ml, preferably 35 mg/ml to 3.5 g/ml.

The thus obtained aqueous solution containing ebselen and cyclodextrin can also be used directly as an injection or an intravenous drip infusion if the solution is produced under aseptic conditions, or the resulting aqueous solution may be sterilized as necessary by such means as filtration or heat sterilization as described above, followed by introduction thereof into a syringe, a vial or an ampoule depending on the case, to give the desired injection or intravenous drip infusion.

For an aseptic dried pharmaceutical preparation produced by introducing the dried product described above into a vial or an ampoule, an aseptic, aqueous solvent such as distilled water for injection can be introduced into its vessel to give the desired injection or intravenous drip infusion.

### BEST MODE FOR CARRYING OUT THE INVENTION

### EXAMPLE 1

10 mg ebselen was dissolved in 2 ml ethanol to give a transparent solution. Separately, 700 mg Captisol^{TM} (referred to hereinafter as SBE7-β-CyD, Cydex Co., Ltd.) was dissolved in 1.2 ml water to give a transparent solution. The respective solutions were placed in a 20-ml egg-plant type flask and confirmed to be transparent and uniform, and then the mixture was dried under reduced pressure for 1 hour in a water bath at 70 °C. Then, the sample was dried for about 12 hours under reduced pressure in a desiccator to give white powder. Purified water was added thereto to adjust the total volume to 0.33 ml, whereby an aqueous, transparent ebselen (30 mg/ml ebselen) solution in which ebselen had been completely solubilized was obtained (ebselen: SBE7-β-CyD = 1 : 10, molar ratio). When the aqueous solution was left for 2 days, no precipitation of crystals was recognized and a transparent aqueous solution was observed.

### EXAMPLE 2

An aqueous solution of ebselen was obtained in the same manner as in Example 1 except that 1543 mg Celdex HP-β-CD_{TM} (with a degree of substitution of 4.6, hereinafter referred to as HP-β-CyD, Nippon Shokuhin Kako Co., Ltd.) (molar ratio, 1 : 30) was used in place of 700 mg SBE 7-β-CyD. When the aqueous solution was left for 2 days, no precipitation of crystals was recognized and a transparent aqueous solution was observed.

### EXAMPLE 3

An aqueous solution of ebselen was obtained in the same manner as in Example 1 except that a methanol-chloroform mixed solvent (2 ml + 1 ml) was used in place of ethanol, and 1 ml purified water was used in place of 1.2 ml purified water. When the aqueous solution was left for 2 days, no precipitation of crystals was recognized and a transparent aqueous solution was observed.

### EXAMPLE 4

An aqueous solution of ebselen was obtained in the same manner as in Example 2 except that a methanol-chloroform mixed solvent (2 ml + 1 ml) was used in place of ethanol, and 1 ml purified water was used in place of 1.2 ml purified water. When the aqueous solution was left for 2 days, no precipitation of crystals was recognized and a transparent aqueous solution was observed.

### EXAMPLE 5

An aqueous solution of ebselen was obtained in the same manner as in Example 1 except that the proportion of ebselen : SBE 7-β-CyD was changed from 1 : 10 (molar ratio) to 1 : 7.8 (molar ratio). When the aqueous solution was left for 2 days, no precipitation of crystals was recognized and a transparent aqueous solution was observed.

### EXAMPLE 6

An aqueous solution of ebselen was obtained in the same manner as in Example 3 except that the proportion of ebselen : SBE 7-β-CyD was changed from 1 : 10 (molar ratio) to 1 : 7.8 (molar ratio) . When the aqueous solution was left for 2 days, no precipitation of crystals was recognized and a transparent aqueous solution was observed.

### COMPARATIVE EXAMPLES

As the methods reported in the literatures as described above, there are the lyophilization method, the kneading method, the solvent evaporation method, the co-precipitation method 1 (using ethanol), the co-precipitation method 2 (using ether) , and the ethanol method.

Any preparation methods were evaluated for production potentiality, and the results are shown below.

### COMPARATIVE EXAMPLE 1

### Lyophilization method

Reference: M. Kurozumi, N. Nambu, T. Nagai, Chem. Pharm. Bull., 23, 3062 (1975)

Ebselen and SBE7-β-CyD were mixed in equal molar amounts (3.64 mM) in a mortar, then a small amount of water was added thereto, the mixture was stirred vigorously for 30 minutes and then dried under reduced pressure for about 12 hours in a desiccator at room temperature. When purified water was added to the resulting powder, the ebselen was precipitated and could not be dissolved transparently.

### COMPARATIVE EXAMPLE 2

### Solvent evaporation method

Reference: M. Tsuruoka, et al., Yakugaku Zasshi (Journal of the Pharmaceutical Society of Japan), 101, 360 (1981)

Ebselen and SBE7-β-CyD were mixed in equal molar amounts (3.64mM) , then added to 3% ammonia water and mixed under stirring. At this time, the chemicals were not dissolved at all. Then, the ammonia water was removed under reduced pressure in a rotary evaporator in a water bath at 35 °C, and then the sample was dried under reduced pressure for about 12 hours in a desiccator at room temperature. When purified water was added to the resulting powder, the ebselen was precipitated and could not be dissolved transparently.

### COMPARATIVE EXAMPLE 3

### Co-precipitation method (1) using ethanol

Reference: M. Tsuruoka, et al., Yakugaku Zasshi, 101, 360 (1981)

Ebselen and SBE7-β-CyD were mixed in equal molar amounts (3.64 mM), then added to 50 % aqueous ethanol, and completely dissolved by heating at 80 °C under stirring. This solution was left at room temperature for 24 hours or more, but precipitates (co-precipitated materials) could not be obtained.

### COMPARATIVE EXAMPLE 4

### Co-precipitation method (2) using ether

Reference: M. Kurozumi, N. Nambu, T. Nagai, Chem. Pharm. Bull., 23, 3062 (1975)

Ebselen (3.64 mM, 1 mg) was dissolved in diethyl ether. Separately, SBE7-β-CyD, 70 g (32.4 mM) was dissolved in purified water. 1 ml each of the solutions were mixed and stirred at room temperature for 24 hours. Then, it was cooled at 2 °C, and precipitates (co-precipitated materials) could not be obtained.

### COMPARATIVE EXAMPLE 5

### Ethanol method (a method of using ethanol as a dissolution assistant)

Reference: J. Pitha, et al., Int. J. Pharm., 80, 253 (1992)

Ebselen and HP-β-CyD were mixed in the 1 : 10 ratio by weight (1 g : 10 g) and then added to 95 % ethanol. This solution was filtered through a membrane filter (GV, 0.22 µm, 25 mmΦ, from Millipore) and further dried for about 12 hours under reduced pressure at room temperature, and the resulting residues were dissolved in purified water and filtered again through a membrane filter (GV, 0.22 µm, 25 mmΦ, Millipore) . Formation of precipitates (ebselen) was observed with time. This solution was lyophilized (RL-20MB model, Kyowa Shinku), and when 1 ml purified water was added to the resulting lyophilized product, the ebselen was precipitated and not dissolved transparently.

### COMPARATIVE EXAMPLE 6

A test was conducted in the same manner as in Comparative Example 5 except that 75 % ethanol was used in place of 95 % ethanol used in Comparative Example 5. As a result, the ebselen was precipitated and not dissolved transparently.

### COMPARATIVE EXAMPLE 7

SBE7-β-CyD was used in place of HP-β-CyD used in Comparative Example 5, and in the same procedure as in Comparative Example 5, the sample was added to 95 % or 75 % ethanol, but the final aqueous solution was cloudy in which precipitation of CyD was recognized. Comparison between the Examples and additional Comparative Examples

The method described in Examples 1 and 2 is referred to as ED method and the method described in Examples 3 and 4 is referred to as MDC method, and the results of these methods, along with those of the preparation method in Comparative Example 4 or 5, are shown in the Table below.

**Table 1**

| [Molar ratio means the molar ratio of ebselen : cyclodextrin (CyD)] | | | | | | |
|---|---|---|---|---|---|---|
| **Type of CyD** | **ED method** | **Molar ratio** | **MDC method** | **Molar ratio** | **Preparation method in Comparative Example 4** | **Preparation method in Comparative Example 5** |
| **HP-β-CyD** | ○ | **1 : 30** | ○ | **1 : 30** | **not producible** | **not producible** |
| **SBE7-β-CyD** | ○ | **1 : 7.8** | ○ | **1 : 7.8** | **not producible** | **not producible** |
| "○" means that a transparent aqueous solution in which ebselen had been completely solubilized could be obtained. | | | | | | |

### INDUSTRIAL APPLICABILITY

The aqueous solution according to the present invention is an aqueous solution having a sufficient amount of ebselen dissolved therein and can be used as a first transparent aqueous injection of this compound.

## Claims

1. An aqueous liquid medicine comprising ebselen and cyclodextrin.

2. An aqueous solution comprising ebselen and cyclodextrin.

3. The aqueous solution according to claim 2, wherein the cyclodextrin is a β-cyclodextrin sulfobutyl ether sodium salt or hydroxypropyl-β-cyclodextrin.

4. The aqueous solution according to claim 2 or 3, wherein the molar ratio of cyclodextrin and ebselen is from 1 : 2 to 1 : 50.

5. An injection comprising an aqueous solution containing ebselen and cyclodextrin.

6. The injection according to claim 5, wherein the cyclodextrin is a β-cyclodextrin sulfobutyl ether sodium salt or hydroxypropyl-β-cyclodextrin.

7. An intravenous drip infusion comprising an aqueous solution containing ebselen and cyclodextrin.

8. The intravenous drip infusion according to claim 7, wherein the cyclodextrin is a β-cyclodextrin sulfobutyl ether sodium salt or hydroxypropyl-β-cyclodextrin.

9. A process for producing an aqueous solution containing ebselen and cyclodextrin, which comprises dissolving ebselen in a water-miscible organic solvent while separately dissolving cyclodextrin in a water solvent, mixing both the solutions, then drying the mixture, and mixing the resulting dried product with a water solvent.

10. The process according to claim 9, wherein the water-miscible organic solvent is ethanol, methanol, or a mixture of methanol and chloroform.

11. A dried preparation comprising ebselen and cyclodextrin.

12. The dried preparation according to claim 11, wherein the cyclodextrin is a β-cyclodextrin sulfobutyl ether sodium salt or hydroxypropyl-β-cyclodextrin.

13. A process for producing a solution containing ebselen and cyclodextrin, which comprises dissolving ebselen in a water-miscible organic solvent while separately dissolving cyclodextrin in a water solvent, and mixing both the solutions.
